# EUROPÄISCHE PATENTANMELDUNG

(11) **EP 1 240 864 A1**
(43) Veröffentlichungstag der Anmeldung: **18.09.2002**
(21) Anmeldenummer: 01105431.9
(22) Anmeldetag: 13.03.2001
(51) Int. Cl.: A61B 5/00, A61B 5/0488

(54) **Vorrichtung und Verfahren zum selektiven Training von Muskeln**

(71) Anmelder: Wiltronic AG, 9435 Heerbrugg (CH)
(72) Erfinder: Nagel, Helmut, 6973 Hoechst (AT)
(74) Vertreter: Kaminski, Susanne

(57) **Zusammenfassung**

Vorrichtung und Verfahren zum selektiven Training mindestens eines ausgewählten Muskels mit mindestens zwei Signalaufnehmern (2;3), Mitteln zur Zuordnung daraus erhaltener Informationen zu den jeweiligen Muskeln (5) und zu vorgegebenen, skalierbaren Werten (6) und Mitteln zur Darstellung (7;8) dieser Werte.

Zu der Vorrichtung gehörende, abnehmbare Schutzhülle für eine Elektrode und Vorrichtung zum Tragen und Positionieren mindestens eines Signalaufnehmers.

Das selektive Training mindestens eines ausgewählten Muskels bewirkt vorzugsweise die Verformung einer geometrischen Figur, die durch eine Darstellung der Werte von Anspannungszuständen mehrerer Muskeln gegeben ist, in Hinsicht auf eine definierte Idealfigur.

## Beschreibung

Die Erfindung betrifft eine Vorrichtung zum selektiven Training mindestens eines ausgewählten Muskels nach dem Oberbegriff des Anspruchs 1, ein Verfahren zum Betreiben einer solchen Vorrichtung nach dem Oberbegriff des Anspruchs 10, sowie eine abnehmbare Schutzhülle für eine Elektrode nach Anspruch 8 und eine Vorrichtung zum Tragen und Positionieren mindestens eines Signalaufnehmers nach Anspruch 9.

Es sind Vorrichtungen und Verfahren zum Training von Muskeln bekannt, bei denen während des Trainings in Echtzeit eine Messung von Muskelaktivitäten oder davon abhängender anderer Körperaktivitäten erfolgt und als Basis für die Trainingsgestaltung verwendet wird.

Mit der Methode des Körpersystem-Kontroll-Trainings oder auch Biofeedback wird versucht, in die körperlichen Vorgänge einzugreifen. Diese besondere Form des Lernens wird mittlerweile in vielen Bereichen angewendet, die Bezüge zur Körperwahrnehmung, Selbstkontrolle oder Selbstheilung besitzen. Unbewusst oder bewusst ablaufende muskuläre Vorgänge wie z. B. Atemfrequenz, Herzfrequenz oder Muskelspannung werden mit entsprechenden Geräten gemessen und an akustische, taktile oder optische Darstellungen gebunden.

Die mit der mechanischen Tätigkeit der Körpermuskulatur verbundene elektrische Aktivität ruft an der Körperoberfläche messbare Spannungsimpulse hervor, die beispielsweise durch Elektromyographie (EMG) über Haut- oder Nadelelektroden gemessen werden können. Andere Methoden einer Signalaufnahme sind taktile Verfahren, die z.B. die Veränderung oder Bewegung der Hautoberfläche messen, oder thermographische Verfahren, die eine Veränderung der Körpertemperatur infolge der Muskelaktivität aufnehmen.

Die Darstellung erfolgt zumeist durch einen Computerbildschirm, einen Vibrationsgeber oder einen Lautsprecher. Der Übende erhält während des Trainings eine unmittelbare Information über die adressierten Muskeln und der Grad der Muskelanspannung wird erlebbar gemacht. Im weiteren Verlauf eines Trainings lernt er, seine muskelbezogenen Körpervorgänge willentlich zu kontrollieren.

Derartige Verfahren gehen also über eine blosse Messung und Rückmeldung von Muskelspannungen hinaus, da im Zuge des Körpersystem-Kontroll-Trainings eine gezielte Selbststeuerung der ursprünglich nicht oder nicht hinreichend differenziert wahrgenommenen Muskelfunktionen angestrebt wird. Durch diese Selbststeuerung wird es dem Übenden prinzipiell möglich, gezielt bestimmte Muskeln trainieren zu können.

Anwendungsbereiche dieser Methode können beispielsweise das Training spezieller Bewegungsabläufe im Sport, die Rehabilitation nach Sportverletzungen oder aber auch die Stärkung spezieller Muskelfunktionen zur Förderung oder Vermeidung von Körperfunktionen, z.B. von Inkontinenz, sein.

Aus der deutschen Patentschrift DE 197 45 508 C2 sind ein Verfahren und eine Vorrichtung bekannt, bei denen ein Nutzer individuelle Entspannungstechniken durch die Beeinflussung fortlaufend gemessener Biosignale seines Körpers erlernt. Durch eine Zusammenführung des eigenen Signals mit dem eines anderen Nutzers und deren Vergleich können gezielt Konkurrenzsituationen erzeugt werden, die einen Entspannungsvorgang unter Wettkampfbedingungen trainierbar macht.

Die Patentschrift US 4 170 225 beschreibt eine tragbare Biofeedback-Vorrichtung zur Aufnahme und Darstellung von Muskelaktivitäten eines durch Elektroden erfassten Bereichs mit visueller und akustischer Anzeige. Die Darstellung erfolgt mittels lichtemittierender Dioden bzw. mittels eines Ohrhörers.

Die deutsche Offenlegungsschrift DE 44 41 267 A1 beinhaltet ein Trainingsgerät zur Behebung der Inkontinenz beim Menschen. Durch das Aufbringen von Elektroden auf unterschiedliche Bereiche des Körpers werden die Signale der dort befindlichen Muskeln aufgenommen und durch zwei Anzeigen aus hintereinander angeordneten Leuchtdioden vergleichbar dargestellt. Eine trainierende Person kann die Anspannung der Bauchdeckenmuskulatur dadurch reduzieren, dass der Unterschied der beiden Anzeigen maximiert wird. Nachteilig ist jedoch die nicht verknüpfte Darstellung der Anspannungszustände, die insbesondere ein Training von mehr als zwei Muskelbereichen aufgrund der mangelnden Übersichtlichkeit von mehreren Reihen aus Leuchtanzeigen schwierig macht. Auch eignet sich die Vorrichtung nur sehr bedingt für einen längerfristigen Gebrauch oder die Nutzung durch Kinder, da die abstrakte Anzeige eine nur geringe Motivationskraft besitzt.

Aus der Patentschrift US 5 423 329 ist ein Verfahren zur Behandlung von Harn-Inkontinenz bekannt, bei dem die Muskelaktivität myographisch aufgezeichnet wird und ein Nutzer von Fachpersonal hinsichtlich der Steuerung seiner Muskelaktivitäten unterwiesen wird. Die benötigten Elektroden werden hierbei beiderseitig des Anus, am Unterleib und am Oberschenkel angebracht. Die Darstellung der erhaltenen Signale erfolgt auf einem Monitor in Form von zwei Kurven für Beckenund Unterleibs-Muskulatur, die die Höhe der Anspannung in Zeitabhängigkeit darstellen. Auch hier bietet die abstrakte Darstellungsweise der nicht miteinander verknüpften Anspannungszustände wenig Anschaulichkeit und eine geringe Motivationswirkung. Eine Trennung der Signale verschiedener Muskeln mit gemeinsamer, verknüpfter Darstellung zur selektiven Adressierung einzelner Muskeln erfolgt nicht.

Dem Stand der Technik entsprechen somit Verfahren und Vorrichtungen, die meist über einen oder mehrere Signalaufnehmer zum Erhalt von Signalen und daraus abgeleiteten Informationen über die Anspannung eines Bereichs von Muskeln verfügen. Aus den zumeist elektrischen Signalen der Signalaufnehmer werden Informationen abgeleitet, deren Darstellung dann meist in Form von optischen Leuchtanzeigen oder Kurvenverläufen erfolgt. Der Benutzer einer Vorrichtung kann dann durch Veränderung der Muskelanspannung die dargestellten Werte verändern.

Ungelöste Probleme bestehen jedoch bei der Handhabbarkeit der Vorrichtungen, insbesondere bei der leicht zu begreifenden Darstellung der Muskelaktivitäten, und der Auflösung von Aktivitäten einzelner, ausgewählter Muskeln.

Die bei Vorrichtungen des Standes der Technik verwendeten Darstellungsweisen sind meist sehr technisch und abstrakt gehalten, z.B. erfolgt die Darstellung durch mehrere Reihen von Leuchtdioden oder in Form von Diagrammen oder Kurvenverläufen. Diese Darstellungsformen erschweren die gleichzeitige Anzeige von mehr als zwei Muskelaktivitäten. Des weiteren werden die dargestellten Informationen nicht oder nicht hinreichend verknüpft präsentiert.

So müssen bei der Darstellung der Muskelaktivitäten in Form von Leuchtdioden mehrere Anzeigen gleichzeitig beobachtet und ständig miteinander verglichen werden. Bei einer Darstellung in Kurvenform müssen die Verläufe von zwei Kurven ständig miteinander verglichen werden. Neben der schon für erwachsene Nutzer schwierigen Aufgabe versagt diese Darstellungsweise bei Anwendungen für Kinder oder Kleinkinder vollständig.

Eine Signalaufnahme der zu trainierenden Muskeln erfolgt zumeist durch Anbringung eines einzelnen Signalaufnehmers im Bereich des zu trainierenden Muskels, wie z.B. durch Klebeelektroden, Anal-/Vaginalsonden oder Nadelelektroden. Während des Trainings wird somit zwar eine An- und Entspannung des gesamten Bereichs erfasst und ein prinzipieller Trainingserfolg möglich, eine gezielte Aufnahme von Signalen für einen einzigen Muskel wird mit diesen Vorrichtungen jedoch nicht geleistet, so dass das Training suboptimal erfolgt und die höchstmögliche Differenzierung bei der Muskelan- und - entspannung nicht zu erreichen ist.

Ausserdem ist bei elektromyographischer Signalaufnahme die aufgezeichnete Amplitude umso grösser, je näher die aktivierten Muskelfasern an den Elektroden liegen und je mehr Fasern zum Signal beitragen. Dadurch kann es vorkommen, dass die Signale falsch zugeordnet werden und statt des eigentlich zu trainierenden Muskels ein näher an der Elektrode liegender Muskel und/oder ein Muskel mit einer grösseren Anzahl von aktivierten Fasern aufgenommen und damit trainiert wird.

Die technische Aufgabe der vorliegenden Erfindung besteht in der Bereitstellung eines Verfahrens und einer Vorrichtung, die eine differenzierende Aufnahme von Aktivitäten verschiedener Muskeln und eine deren Werte in einer Anzeige verknüpfende Darstellung gewährleistet, mit ergänzender Schutzhülle und einer Vorrichtung zum Tragen und Positionieren von Signalaufnehmern.

Diese Aufgabe wird erfindungsgemäss durch die kennzeichnenden Merkmale der Ansprüche 1, 8, 9 bzw. 10 gelöst. Vorteilhafte und alternative Ausgestaltungen und Weiterbildungen der Vorrichtungen und des Verfahrens ergeben sich aus den Merkmalen der Unteransprüche.

Die erfindungsgemässe Vorrichtung beinhaltet Signalaufnehmer zur Aufnahme von Signalen, Mittel zur Zuordnung von in den Signalen enthaltenen Information zu den jeweiligen Muskeln und zu vorgegebenen, skalierbaren Werten und Mittel zur Darstellung der wenigstens zwei Werte. Die anwendungsspezifische Beeinflussung von mindestens einem der Muskeln durch einen Anwender der Vorrichtung wird über die Mittel zur Darstellung überprüfbar gestaltet.

Die Signalaufnehmer können so gestaltet sein, dass sie vorwiegend oder ausschliesslich Signale nur eines zu trainierenden Muskels oder einer Muskelgruppe oder aber eine Vielzahl von Signalen unterschiedlicher Muskeln aufnehmen, deren Trennung später auf anderem Weg erfolgt. Die aufgenommenen Signale beinhalten somit Informationen über nur einen Muskel oder aber eine Mischung von Informationen über die Anspannungszustände verschiedener Muskeln. Beispielsweise kann je nach Lage der zu trainierenden Muskeln ein ausreichendes Signal durch Plazierung einer einzelnen Elektrode auf der Körperoberfläche in der Nähe des Muskels oder durch eine Mehrzahl von Elektroden, aus deren Signalen der Anspannungszustand des Muskels gefiltert werden muss, erhalten werden. Eine Anbringung der Elektrode im Inneren des Körpers oder in einem Körperhohlraum ergibt einen geringen Abstand zum aufzunehmenden Muskel, was in einigen Fällen zwar wünschenswert, aber nicht immer problemlos zu realisieren ist.

Der Anspannungszustand der Muskeln kann von den Signalaufnehmern elektromyographisch, taktil, thermographisch oder auf jede andere geeignete Weise erfasst werden. So kann beispielsweise auch sonographisch, z.B. mit Ultraschall, der Kontraktionszustand bzw. die Differenz zwischen zwei Zuständen gemessen werden. Eine verwendbare Methode benutzt die Messung des Hautwiderstandes bzw. die Impedanz als Mass für eine Muskelanspannung. Als weitere Signalaufnehmer können drucksensitive Sonden, beispielsweise Ballonsonden, verwendet werden, die sich besonders für ein Training ringförmiger Muskeln, wie z.B. des Schliessmuskels eignen.

Nach der Aufnahme der Signale werden die Informationen zur weiteren Verarbeitung den jeweils hinsichtlich ihrer Aktivität darzustellenden Muskeln zugeordnet. Dies erfolgt durch die Mittel zur Zuordnung der Information entweder nach einer nach Muskeln getrennten und selektiven Signalaufnahme, bei der gegebenenfalls enthaltene Anteile anderer Muskeln gefiltert oder abgetrennt werden können, oder bei der gleichzeitigen Aufnahme von Signalen bzw. Informationen verschiedener Muskeln durch die Signalaufnehmer auf algorithmischem oder schaltungstechnischem Weg. Zur Zuordnung kann dabei beispielsweise eine elektronische Schaltung oder ein Rechner verwendet werden.

Durch die selben oder weitere Komponenten erfolgt danach eine Zuordnung der nun getrennten muskelspezifischen Information zu vorgegebenen und skalierbaren Werten. Eine Skalierung der Werte gewährleistet, dass auch bei nur geringer Muskelanspannung noch erkennbare Werte für die Darstellung zur Verfügung stehen. Diese Situation wird insbesondere bei einem Training der Fall sein, bei dem ein Muskel bei gleichzeitiger Entspannung anderer, benachbarter Muskeln trainiert werden soll. Mit zunehmendem Trainingserfolg wird dabei aufgrund verbesserter Entspannung ein Signal zunehmend schwächer, so dass zur Gewährleistung eines weiteren Trainingsfortschrittes der dem Muskel zugeordnete Wert überhöht dargestellt werden kann, da nur so noch geringe Veränderungen des Anspannungszustandes wahrnehmbar sind.

Die Zuordnung der Werte kann dabei mit einer Umrechnung auf der Basis des gemessenen Rohwertes erfolgen oder aber beispielsweise auch auf der Basis abgeleiteter Grössen erfolgen. So kann z.B. die Differenz aus aktuellem Anspannungszustand und dem vorher erreichten Entspannungszustand dargestellt werden. Die dargestellten Werte beziehen sich damit nicht auf Absolutgrössen sondern betrachten das Entspannungs- bzw. Anspannungsvermögen.

Die Werte der Anspannungszustände werden über die Mittel zur Darstellung so dargestellt, dass die anwendungsspezifische Beeinflussung von mindestens einem der Muskeln durch einen Anwender der Vorrichtung überprüfbar wird. Diese Mittel können insbesondere eine optisch-visuelle, eine akustische und/oder eine taktile Anzeige aufweisen. Diese Anzeigen können sich gegenseitig ersetzen, was beispielsweise eine Verwendung durch sehbehinderte oder blinde Anwender ermöglicht, oder im Wechsel oder auch in Kombination verwendet werden, um z.B. Freizeitbeschäftigungen, beispielsweise Lesen oder Fernsehen, während des Trainings berücksichtigen zu können.

Auch können mit zunehmendem Trainingsfortschritt die Darstellungsformen gewechselt werden. So kann es für einen bereits relativ gut trainierten Nutzer hinreichend sein, die unbeabsichtigte Anspannung von peripheren und nicht zu trainierenden Muskeln lediglich in Form eines Warnsignals akustisch anzuzeigen, während die Anspannung des zu trainierenden Muskels durch eine Variation der Tonhöhe angezeigt wird.

Vorzugsweise erfolgt die Darstellung, zumindest zu Beginn des Trainings, optisch-visuell. Auf einem Bildschirm, beispielsweise in Form eines LCD-Displays oder einer Kathodenstrahlröhre, können die Werte für unterschiedliche Muskeln in verknüpfter Form dargestellt werden, z.B. als geometrische Figur oder als Computerspiel.

Eine Verknüpfung der Anspannungszustände verschiedener Muskeln in Form einer gleichzeitigen Darstellung verursacht bei mehr als zwei Muskeln Probleme, da eine Darstellung der zugeordneten Werte nur in einem Diagramm mit maximal zwei Achsen erfolgen kann. Bereits eine perspektivische Darstellung mit drei Achsen ist nicht einfach zu realisieren, da an das Display spezielle Anforderungen gestellt werden müssen und die abgebildete Darstellung schnell unklar werden kann.

Die Einbeziehung von mehr als zwei Werten in eine graphische Darstellung geschieht besonders anschaulich dadurch, dass die Muskeln jeweils einzelnen Eigenschaften einer Figur, wie z.B. geometrischen Formparametern (beispielsweise Skalierungsfaktor, Dimension von Achsen, Zahl von Ecken), geometrischen Lageparametern (beispielsweise Neigung gegenüber einer Achse), der Farbe, der Strukturierung (beispielsweise Schraffur), der Periodizität (beispielsweise Sichtbarkeitsdauer, Rotationsgeschwindigkeit) oder der Intensität der Darstellung (beispielsweise Helligkeit), zugeordnet werden.

So kann beispielsweise ein Vieleck durch Entspannen des betreffenden Muskels in der Zahl seiner Ecken reduziert werden. Der zu erreichende Zustand maximaler Entspannung kann dann beispielsweise durch ein Quadrat oder einen Kreis, z.B. mit den hinsichtlich abnehmender Eckenzahlen zu durchlaufenden Formen Vieleck-Linse-Tropfen-Kreis, dargestellt werden.

Eine Darstellung der in der jeweiligen oder einer früheren Trainingssitzung erreichten Bestleistung kann beispielsweise auf einer Anzeige durch Schleppzeiger erfolgen.

Eine leicht begreifbare Ausführungsform der graphischen Darstellung stellt die Darstellung von zwei oder mehr Muskeln in Form einer Ellipse dar. Dabei werden beispielsweise die ersten beiden Muskeln jeweils einer der beiden Hauptachsen und ein dritter Muskel dem Neigungswinkel einer der Hauptachsen gegenüber der Senkrechten zugeordnet. Besteht nun das Trainingsziel in der Belastung eines Muskels bei gleichzeitiger Entspannung der beiden anderen Muskeln, so kann als Aufgabe eine Verformung der Ellipse vorgegeben werden. Das anzustrebende Idealbild kann dann beispielsweise als senkrechter Strich vorgegeben werden. Das Entspannen eines der Muskeln führt zur Verringerung einer der Hauptachsen, so dass die Ellipse zu einem Strich wird. Das Entspannen des anderen Muskels kippt den Strich oder die Ellipse in die Senkrechte. Die so dargestellte Aufgabe kann auch von Kindern gelöst werden, ausserdem bietet sie aufgrund entfallender Anzeigen die Möglichkeit, bei einer entsprechend deutlichen Darstellung, dieses Trainingsverfahren auch Sehbehinderten zugänglich zu machen.

Die graphische Darstellung einer Figur kann dabei jedoch auch grundsätzlich durch weitere Elemente, z.B. in Form einer Absolutwertanzeige oder der parallelen Angabe der Rohwerte, ergänzt werden.

Für Kinder kann die dargestellte Figur darüber hinaus auch als Märchen-, Tier- oder Comicfigur gewählt werden, die beispielsweise in ihrer Grösse und Farbe zu verändern ist. Eine Ergänzung bietet die Kombination mit akustischen Anzeigen wie z.B. das Erzeugen eines Tones oder das Abspielen einer Melodie beim Erreichen eines vorgewählten Sollwertes.

Eine weitere Ausführungsform stellt das durch die Muskelanspannung gesteuerte Spielen eines Computerspieles dar, bei dem beispielsweise eine vorgegebene Aufgabe zu lösen ist.

Die akustische Darstellung kann durch ein Audiosignal erfolgen, das über Kopfhörer oder Lautsprecher wahrgenommen und beispielsweise in der Tonhöhe, der Lautstärke oder seiner Periodizität variiert wird. Als ein taktiles Mittel zur Darstellung können z.B. eine oder mehrere vibrierende Auflagen verwendet werden, die beispielsweise jeweils einem Muskel zugeordnet sind und deren Aktivierung bzw. Vibrationsintensität einem Anspannungszustand des speziellen Muskels entspricht. Durch die Verwendung taktiler Mittel oder eines Kopf- oder Ohrhörers lässt sich ein für die Umgebung nicht wahrnehmbares Training realisieren.

Die Verwendung drahtloser Übertragungsmittel zwischen einzelnen Komponenten der Vorrichtung erlaubt eine gesteigerte Mobilität des Anwenders. So kann durch eine apparative Dreiteilung in am Körper zu tragende Signalaufnehmer, fest plazierte Auswerteeinheit und Ohrhöher mit einer drahtlose Übertragung zwischen den Komponenten eine Beweglichkeit beim Training erreicht werden, die es beispielsweise ermöglicht, zeitgleich Arbeiten zu erledigen.

Die erfindungsgemässe Vorrichtung und das Verfahren werden nachfolgend anhand von in der Zeichnung schematisch dargestellten Ausführungsbeispielen rein beispielhaft näher beschrieben. Im einzelnen zeigen
- Fig. 1: eine schematische Gesamtdarstellung einer erfindungsgemässen Vorrichtung,
- Fig. 2: eine mögliche Anbringung von Klebeelektroden auf der Körperoberfläche im Bereich der Brust- und Bauchmuskulatur,
- Fig. 3: die geometrischen Verhältnisse für die beispielhafte gleichzeitige Darstellung von Werten dreier Muskeln auf einer Anzeige,
- Fig. 4: eine beispielhafte Darstellung von Werten dreier Muskeln, die jeweils einen teilweise angespannten Zustand besitzen, auf einer Anzeige,
- Fig. 5: eine beispielhafte Darstellung von Werten dreier Muskeln, bei der ein Muskel einen völlig entspannten Zustand besitzt, auf einer Anzeige,
- Fig. 6: eine beispielhafte Darstellung von Werten dreier Muskeln, bei der ein anderer Muskel einen völlig entspannten Zustand besitzt, auf einer Anzeige,
- Fig. 7: eine beispielhafte Darstellung von Werten dreier Muskeln, bei der sich zwei Muskeln im Zustand völliger Entspannung befinden, wobei der dritte Muskel maximal angespannt ist, auf einer Anzeige.

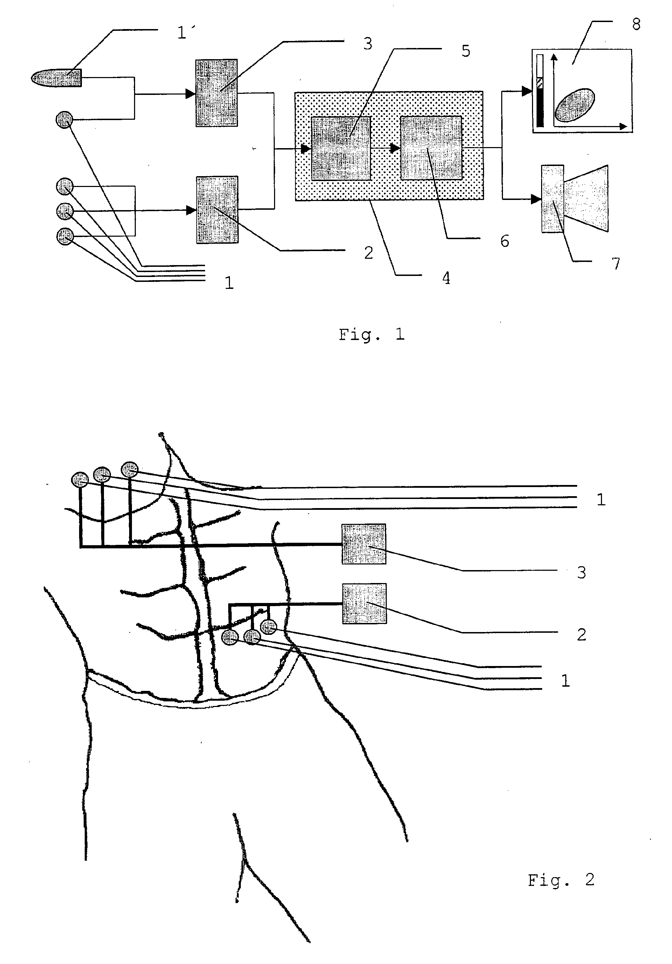

Fig.1 zeigt den schematischen Aufbau einer erfindungsgemässen Vorrichtung mit vier Klebeelektroden 1 und einer Anal-/Vaginalsonde 1'. Den mit der analen oder vaginalen Positionierung verbundenen Hygieneerfordernissen wird durch eine abnehmbare Ein- oder Mehrweghülle der Elektrode Rechnung getragen. Die Hülle kann nach der Trennung von dem Elektrodenkörper entweder entsorgt oder leicht gereinigt bzw. desinfiziert oder sterilisiert werden.

Die durch die Elektroden 1, 1' des ersten Signalaufnehmers 2 und des zweiten Signalaufnehmers 3 aufgenommenen Signale mit den darin beinhalteten Informationen über den Anspannungszustand der Muskeln werden in der Muskel-Zuordnungseinheit 5 getrennt und zugeordnet. Die Zuordnung zu vorgegebenen, skalierbaren Werten erfolgt darauffolgend in der Wert-Zuordnungseinheit 6. Beide Zuordnungseinheiten 5, 6 sind in einer Auswerteeinheit 4 baulich zusammengefasst. Die erhaltenen Werte werden durch ein akustisches Darstellungsmittel 7 und ein visuelles Darstellungsmittel 8 angezeigt.

Fig.2 stellt die beispielhafte Anbringung von Klebeelektroden 1 auf der Körperoberfläche im Bereich der Brustmuskulatur und der Bauchmuskulatur dar. Die Signale werden für die beiden Bereiche durch einen ersten Signalaufnehmer 2 und einen zweiten Signalaufnehmer 3 getrennt aufgenommen. Die Signalaufnahme und -verarbeitung kann eine Differenzierung nach den beiden Bereichen Brust und Bauch beinhalten oder aber durch eine weitere Verarbeitung und Trennung der Signale nach einzelnen Muskeln innerhalb der beiden Bereiche ein muskelspezifisches Training ermöglichen. Eine Positionierung und Fixierung der Elektroden 1 kann durch Aufkleben auf die Körperoberfläche oder auch durch eine Tragevorrichtung, die beispielsweise als Weste ausgestaltet ist, bewirkt werden. Durch eine körpergerecht ausgeformte Tragevorrichtung kann eine leichte, präzise und gleichzeitige Positionierung und Fixierung ohne fremde Hilfe, insbesondere ohne ständige ärztliche Betreuung, erreicht werden.

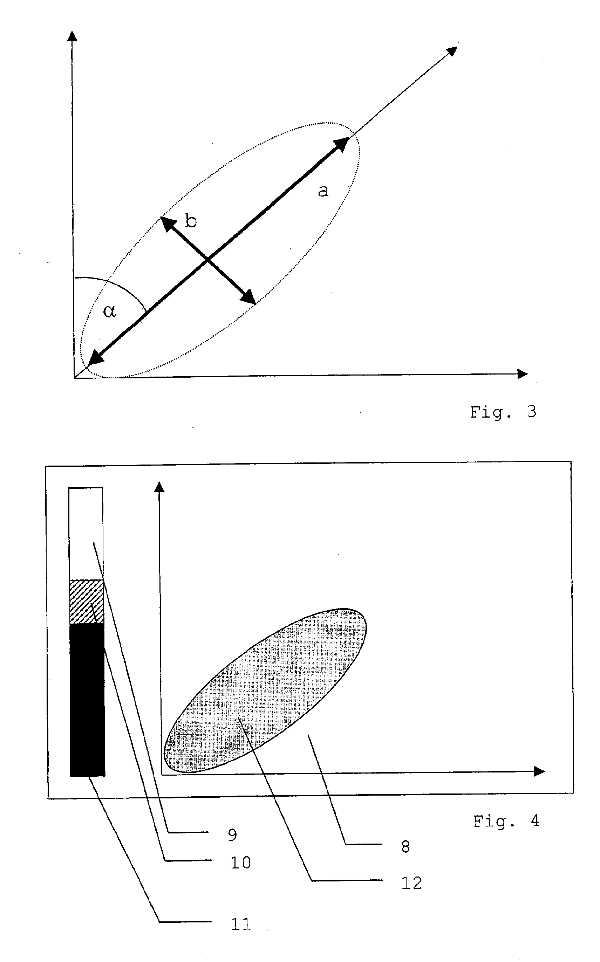

In Fig.3 werden die geometrischen Verhältnisse für eine gleichzeitige Darstellung von Werten dreier Muskeln auf einer Anzeige gezeigt. Als graphische Darstellung wird eine Ellipse gewählt, deren Figureigenschaften Erste Hauptachse a, Zweite Hauptachse b und Neigungswinkel α gegenüber der Senkrechten jeweils den Werten für einen speziellen Muskel entsprechen. Durch eine Veränderung der Muskelanspannung kann nun die Ellipse verformt oder in ihrer Neigung verändert werden. Da die Werte so skaliert sind, dass eine Darstellung auch kleiner Veränderungen möglich ist, sind die Hauptachsen a, b und der Neigungswinkel α nicht absolut vergleichbar. Das Ziel des Trainings kann nun darin bestehen, ausschliesslich den der Hauptachse b zugeordneten Muskel anzuspannen, wobei die beiden anderen Muskeln vollständig entspannt werden sollen, so dass die geometrische Figur 12 zu einem senkrechten Strich verformt wird.

Fig.4 zeigt eine Darstellung der in Fig.3 beschriebenen Situation auf einer möglichen Anzeige. Dabei sind alle drei Muskeln teilweise angespannt, die geometrische Figur 12 erscheint ellipsenförmig und gegenüber der Senkrechten geneigt. Auf der linken Seite sind auf einer Absolutwertanzeige 9 der maximale Wert der Muskelanspannung 10 und der aktuelle Wert der Muskelanspannung 11 im Vergleich angegeben.

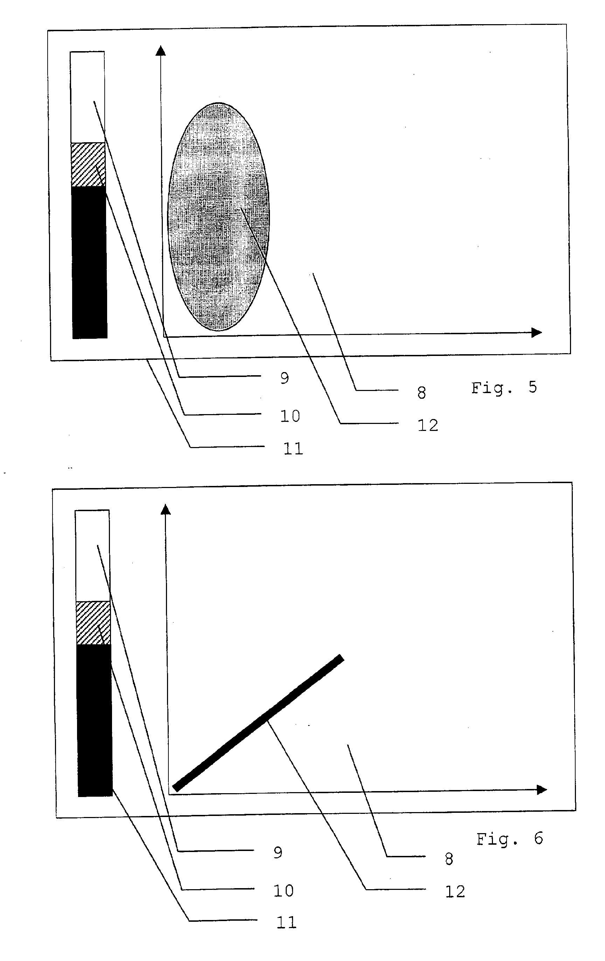

Die Situation bei Entspannung eines der Muskeln wird in Fig.5 gezeigt. Durch das vollständige Entspannen eines Muskels steht die Figur senkrecht, die beiden anderen Muskeln besitzen Spannung, so dass die Ellipsenform der geometrischen Figur 12 erhalten bleibt.

Fig.6 beschreibt die Situation bei vollständiger Entspannung eines anderen der drei Muskeln. Die Neigung und die Ausdehnung der Hauptachse a bleiben erhalten, die Hauptachse b hat den Wert Null, so dass die geometrische Figur 12 zu einer Geraden verformt wird.

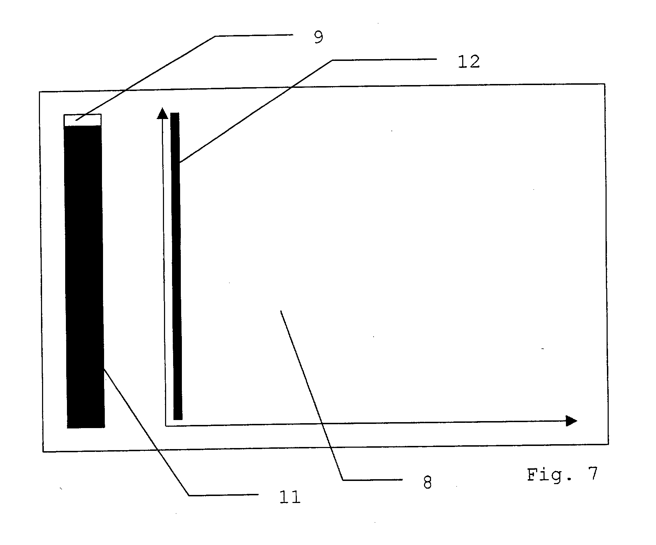

In Fig.7 wird der zu erreichende Zielzustand dargestellt. Der zu trainierende Muskel ist maximal angespannt, wobei der aktuelle Wert der Muskelanspannung 11 den bisherigen maximalen Wert überschreitet, die anderen Muskeln sind vollständig entspannt. Gemäss dem vorgegebenen Idealbild hat die geometrische Figur 12 die Form einer senkrecht stehenden Gerade.

Es versteht sich, dass die dargestellten Figuren eine von vielen Ausführungsformen darstellen und der Fachmann alternative Realisierungsformen, z.B. unter Verwendung anderer Mittel zur Signalaufnahme oder -verarbeitung, ableiten kann.

## Patentansprüche

1. Vorrichtung zum selektiven Training mindestens eines ausgewählten Muskels
mit mindestens zwei Signalaufnehmern (2;3) zur Aufnahme von Signalen, die Informationen über den Anspannungszustand des ausgewählten und mindestens eines weiteren Muskels beinhalten,
mit Mitteln zur Zuordnung der Information zu den jeweiligen Muskeln (5) und zu vorgegebenen, skalierbaren Werten (6),
wobei die anwendungsspezifische Beeinflussung von mindestens einem der Muskeln durch einen Anwender der Vorrichtung über Mittel zur Darstellung (7;8) überprüfbar wird,
**dadurch gekennzeichnet, dass**
die Mittel zur Darstellung (7;8) so gestaltet sind, dass eine verknüpfte Darstellung der wenigstens zwei Werte erfolgt.

2. Vorrichtung nach Anspruch 1,
**dadurch gekennzeichnet, dass**
die Signalaufnehmer (2;3) mindestens eine Elektrode (1;1') umfassen, welche auf die Körperoberfläche aufbringbar und/oder anal oder vaginal in den Körper einführbar ist.

3. Vorrichtung nach einem der vorangehenden Ansprüche,
**dadurch gekennzeichnet, dass**
die Signalaufnehmer (2;3) die Signale auf mindestens eine der folgenden Weisen aufnehmen
• Elektromyographisch,
• Taktil,
• Drucksensitiv,
• Sonographisch,
• Thermographisch,
• mittels Impedanzmessung.

4. Vorrichtung nach einem der vorangehenden Ansprüche,
**dadurch gekennzeichnet, dass**
die Signalaufnehmer (2;3) oder die Mittel zur Zuordnung der Informationen zu den jeweiligen Muskeln (5) mindestens einer der folgenden Funktionsweisen zur Trennung der Informationen aufweisen
- muskelspezifische, separate Aufnahme von Signalen des ausgewählten Muskels
- Zuordnung der in den Signalen beinhalteten Information zu den Muskeln auf algorithmischem Weg oder durch eine elektronische Schaltung.

5. Vorrichtung nach einem der vorangehenden Ansprüche,
**dadurch gekennzeichnet, dass**
die Mittel zur Darstellung (7;8) mindestens eine optisch-visuelle Anzeige (8), insbesondere eine gleichzeitige graphische Darstellung des Anspannungszustands mehrerer Muskeln, z.B. auf einem Display, und/oder eine akustische und/oder eine taktile Anzeige aufweisen.

6. Vorrichtung nach einem der vorangehenden Ansprüche,
**dadurch gekennzeichnet, dass**
die Mittel zur Darstellung (7;8) einen Schleppzeiger zur Darstellung der Bestleistung einer aktuellen Trainingssitzung aufweisen.

7. Vorrichtung nach einem der vorangehenden Ansprüche,
**dadurch gekennzeichnet, dass**
Mittel zur drahtlosen Übertragung von Signalen und/oder Informationen und/oder Werten zwischen Bestandteilen der Vorrichtung beinhaltet sind.

8. Abnehmbare Schutzhülle, insbesondere Einweghülle, für eine Elektrode (1:1') einer Vorrichtung nach einem der Ansprüche 2 bis 7.

9. Vorrichtung zum Tragen und Positionieren mindestens eines Signalaufnehmers (2;3) einer Vorrichtung nach einem der Ansprüche 2 bis 7 am Körper.

10. Verfahren zum Betreiben einer Vorrichtung nach einem der Ansprüche 1 bis 7,
**dadurch gekennzeichnet, dass**
wenigstens folgende Schritte durchgeführt werden
• Aufnehmen von Signalen, die Informationen beinhalten, die kennzeichnend sind für einen Anspannungszustand eines ausgewählten und mindestens eines weiteren Muskels, mittels Signalaufnehmern (2;3) zur Aufnahme von Signalen, die Informationen über den Anspannungszustand des ausgewählten und mindestens eines weiteren Muskels beinhalten,
• Ableiten von Informationen, die jeweils den Anspannungszustand der mindestens zwei unterschiedlichen Muskeln beschreiben, aus den Signalen,
• Zuordnen von skalierbaren Werten, die den Anspannungszustand der mindestens zwei unterschiedlichen Muskeln repräsentieren, zu den Informationen, die jeweils den Anspannungszustand der mindestens zwei unterschiedlichen Muskeln beschreiben,
• Darstellen der skalierbaren Werte derart, dass eine Veränderung des Anspannungszustandes mindestens eines Muskels den diesem Anspannungszustand zugeordneten skalierbaren Wert gegenüber dem mindestens einen verbleibenden skalierbaren Wert mindestens eines weiteren Muskels vermindert oder vergrössert.

11. Verfahren nach Anspruch 10,
**dadurch gekennzeichnet, dass**
das Ableiten von Informationen aus den Signalen eine Trennung der Signale
• durch Aufnahme über wenigstens einen für mindestens einen Muskel spezifischen Signalaufnehmern (2;3) und/oder
• auf algorithmischen Weg mit einen Rechner und/oder
• durch eine elektronische Schaltung
bewirkt.

12. Verfahren nach einem der Ansprüche 10 bis 11,
**dadurch gekennzeichnet, dass**
das Darstellen der Werte mittels einer optischvisuellen, insbesondere einer gleichzeitigen graphischen Darstellung der Anspannungszustände mehrerer Muskeln, und/oder akustischen und/oder taktilen Anzeige und/oder in Form eines Computerspieles erfolgt

13. Verfahren nach einem der Ansprüche 10 bis 12,
**dadurch gekennzeichnet, dass**
das Zuordnen der Signale zu den Werten so erfolgt, dass durch eine Skalierung auf eine jeweils geeignete Grössenskala eine vergleichbare Darstellung der Anspannungszustände mehrerer Muskeln erfolgt.

14. Verfahren nach einem der Ansprüche 10 bis 13,
**dadurch gekennzeichnet, dass**
das Darstellen der Werte für die Anspannungszustände mehrerer Muskeln in Form einer Figur (12) erfolgt, der gegebenenfalls ein Idealbild vorgegeben wird, und bei der wenigstens eine der Eigenschaften
• geometrische Formparameter (beispielsweise Skalierungsfaktor, Dimension von Achsen, Zahl von Ecken)
• geometrische Lageparameter (beispielsweise Neigung gegenüber einer Achse)
• Farbe
• Strukturierung (beispielsweise Schraffur)
• Periodizität der Darstellung (beispielsweise Sichtbarkeitsdauer, Rotationsgeschwindigkeit)
• Intensität der Darstellung (beispielsweise Helligkeit)
entsprechend des Anspannungszustandes des jeweiligen Muskels variieren.

15. Verfahren nach Anspruch 14,
**dadurch gekennzeichnet, dass**
die Figur (12) eine Ellipse darstellt, deren Hauptachsen und deren Neigung gegenüber der Senkrechten jeweils den Anspannungszustand eines speziellen Muskels darstellen.

16. Verfahren nach einem der Ansprüche 10 bis 15,
**dadurch gekennzeichnet, dass**
bei dem Darstellen der Werte ein Sollwert vorgegeben wird, der beispielsweise aus einer Differenz zwischen maximaler Muskelan- und Muskelentspannung folgt.
